# EUROPEAN PATENT APPLICATION

(11) **EP 4 470 528 A1**
(43) Date of publication of application: **04.12.2024**
(21) Application number: 23185720.2
(22) Date of filing: 17.07.2023
(51) Int. Cl.: A61K 9/14, A61K 31/593, A61K 47/69

(54) **ACTIVE INGREDIENT COMPLEX AND METHOD OF MAKING THEREOF**

(30) Priority: 31.05.2023 WO PCT/CN2023/097341
(71) Applicant: Haleon US Holdings LLC, Wilmington, DE 19808 (US)
(72) Inventor: Ai, Alex, Weybridge, KT13 0NY (GB); Dai, Yong, Weybridge, KT13 0NY (GB)
(74) Representative: Haleon Patent Department

(57) **Abstract**

A particulate material, a method of making a particulate material, and an application thereof, where the particulate material includes a complex formed from an active ingredient and a complexing agent. The particulate material may include a carrier.

## Description

### TECHNICAL FIELD

The invention is generally related to an active ingredient complex and compositions including the active ingredient complex.

### BACKGROUND

Formulations such as pharmaceuticals, nutraceuticals, nutrition supplements, cosmetics, and agricultural products, can contain an ingredient that in some way contributes to the beneficial effects experienced by consumers of that formulation.

In certain circumstances, it may be difficult to add the ingredient to the formulation in a manner that provides the beneficial effects that the consumer desires. For example, the ingredient may not be readily soluble in a manner needed for the manufacturing process, the ingredient may not be stable in the desired formulation for an extended period of time, or the beneficial effects of the ingredient may not be readily achieved due to the nature of the dosage form. Other inefficiencies may exist due to the nature of the ingredient.

In such cases, it may be necessary to use inefficient methods in preparing the formulation in order to achieve the desired effect. For example, it may be necessary to add an excessive amount of the ingredient to the manufacturing process in order to end up with a formulation that contains an appropriate amount of the ingredient. Or in other cases, the ingredient may be unstable which would require inefficient handling requirements or earlier expiration dates.

It would be desirable to develop a more efficient way of delivering active ingredients to the consumer.

### SUMMARY

A method of making a particulate material, comprising forming a complex from a water-insoluble vitamin and a complexing agent comprising a cyclodextrin, and forming a particulate material from the complex, wherein the particulate material comprises a plurality of particulates and the content uniformity of the plurality of particulates is no greater than 2%.

A particulate material formed according to the method.

A dosage form containing the particulate material.

### BRIEF DESCRIPTION OF THE DRAWINGS

Figure 1 is a flowchart illustrating a method of making Samples in Example 1.
Figure 2 is a graph illustrating the results of a small batch content uniformity test.
Figure 3 is a graph illustrating the results of a large batch content uniformity test.
Figure 4 is a flowchart illustrating a method of making Samples in Example 2.
Figure 5 is a graph illustrating the results of an accelerated stability test.
Figure 6 is a graph illustrating the results of a long term stability test.

### DETAILED DESCRIPTION

Embodiments described herein can be understood more readily by reference to the following detailed description, examples, and figures. Elements, apparatus, and methods described herein, however, are not limited to the specific embodiments presented in the detailed description, examples, and figures. It should be recognized that the exemplary embodiments herein are merely illustrative of the principles of the present invention. Numerous modifications and adaptations will be readily apparent to those of skill in the art without departing from the spirit and scope of the invention.

In addition, all ranges disclosed herein are to be understood to encompass any and all subranges subsumed therein. For example, a stated range of "1.0 to 10.0" should be considered to include any and all subranges beginning with a minimum value of 1.0 or more and ending with a maximum value of 10.0 or less, e.g., 1.0 to 5.3, or 4.7 to 10.0, or 3.6 to 7.9.

All ranges disclosed herein are also to be considered to include the end points of the range, unless expressly stated otherwise. For example, a range of "between 5 and 10" or "5 to 10" or "5-10" should generally be considered to include the end points 5 and 10.

It is further to be understood that the feature or features of one embodiment may generally be applied to other embodiments, even though not specifically described or illustrated in such other embodiments, unless expressly prohibited by this disclosure or the nature of the relevant embodiments. Likewise, compositions and methods described herein can include any combination of features and/or steps described herein not inconsistent with the objectives of the present disclosure. Numerous modifications and/or adaptations of the compositions and methods described herein will be readily apparent to those skilled in the art without departing from the present subject matter.

Unless expressly defined otherwise, all references to weight percentage (wt.%) refer to a weight percentage based on a total weight of the composition.

As used herein, the term "major portion" refers to a portion that is equal to or greater than 30% by weight of a mixture. As used herein, the term "minor portion" refers to a portion that is less than 30% by weight of a mixture.

As used herein, the term "absolute alcohol" or "absolute ethanol" refers to an ethanol containing less than 1% water by weight.

As used herein, an "active ingredient" refers to any component that provides biologically active or other direct effect in the diagnosis, cure, mitigation, treatment, or prevention of disease or to affect the structure or any function of the body of humans or animals.

As used herein, the term "excipient" refers to any component other than the active agent that aids in the functionality of the formulation.

As used herein, the term "water insoluble" refers to a compound that can be dissolved in water only to a concentration of less than 0.1 wt%. As used herein, the terms "slightly soluble" or "slightly water soluble" refer to a compound that can be dissolved in water only to a concentration of 0.1 to 1.0 wt-%. As used herein, the term "water soluble" refers to a compound that can be dissolved in water at a concentration of more than 1 wt%.

### Complex Particulate Material

Disclosed herein is a particulate material comprising a complex, the complex containing an active ingredient and a complexing agent. The particulate material can further include a carrier.

Also disclosed herein is a method of making the particulate material. The method can further include forming a particulate material comprising the complex. In an embodiment, the method can include combining the complex and the carrier to form the particulate material.

The particulate material made according to the method described herein can provide increased content uniformity, increased purity, and increased stability of the active ingredient in a dosage form, particularly when the carrier is used.

### Complexing Agent

As discussed above, a complexing agent may be used in the preparation of the complex. The complexing agent can assist stabilizing or making soluble an active ingredient that on its own is unstable or insoluble.

In an embodiment, the complexing agent can include a cyclic oligosaccharide having a plurality of saccharide units. In a particular embodiment, the complexing agent can include a cyclic oligosaccharide according to Formula 1, where S is a saccharide unit and n is 0, 1, 2, 3, or 4.

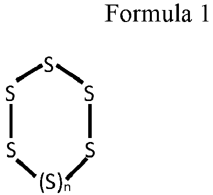

In a particular embodiment, n is 0, 1, 2 or 3. In a more particular embodiment, n is 1, 2, or 3. In a more particular embodiment, n is 2. In an embodiment, the saccharide units are typically linked via the C1 and C4 positions of the saccharide, i.e. each bond is a C1 → C4 bond. Alternative configurations are also possible. In a particular embodiment, each saccharide unit is joined to another saccharide unit by an α-1,4 glycosidic bond.

In an embodiment, each saccharide unit of the plurality of saccharide units can be the same or different than one or more of the plurality of saccharide units. In an embodiment, a saccharide unit can include a substituted or unsubstituted monosaccharide unit. In an embodiment, the monosaccharide unit can include a pentose saccharide or a hexose saccharide and is typically a hexose saccharide. Examples of suitable saccharide units are ribose, xylose, glucose, galactose or mannose or a derivative thereof. In one embodiment each saccharide unit is the same or different and represents glucose or a derivative thereof. In a particular embodiment, the glucose includes a cyclic glucose such as a glucofuranose or a glucopyranose.

The saccharide units may be in either D or L form, or a combination of D and L form saccharide units may be present. Typically, either all saccharide units have D form or all saccharide units have L form. In a particular embodiment, the saccharide unit can include a glucopyranose, such as a D- and L-glucopyranose. In a particular embodiment, a saccharide unit can include a D-glucopyranose.

In an embodiment, a saccharide unit can include a substituted monosaccharide. When S represents a hexose saccharide unit, typically the functional group is present at the primary C6 position. When S represents a pentose saccharide unit, typically the functional group is present at the primary C5 position. In a particular embodiment, the substituted monosaccharide can be a hydroxypropyl substituted monosaccharide. For example, when a monosaccharide unit is D-glucopyranose, the substituted version of that monosaccharide can include a hydroxypropyl-D-glucopyranose.

In a preferred embodiment, the cyclic oligosaccharide can include a cyclodextrin. In an embodiment, the cyclic oligosaccharide can include an α-cyclodextrin, β-cyclodextrin, or a γ-cyclodextrin. In a particular embodiment, the cyclic oligosaccharide includes a α-cyclodextrin. In a more particular embodiment, the cyclic oligosaccharide includes a hydroxypropyl-α-cyclodextrin. In a preferred embodiment, the cyclic oligosaccharide includes a β-cyclodextrin. In a more particular embodiment, the cyclic oligosaccharide includes a hydroxypropyl-β-cyclodextrin. In a particular embodiment, the cyclic oligosaccharide includes a γ-cyclodextrin. In a more particular embodiment, the cyclic oligosaccharide includes a hydroxypropyl-γ-cyclodextrin.

In an embodiment, the complexing agent can be present in the complex in an amount of at least 20 wt%, or at least 30 wt%, or at least 40 wt%, based on a total weight of the complex absent any weight contributed by the carrier. The active ingredient can be present in the complex in an amount of no greater than 65 wt%, or no greater than 60 wt%, or no greater than 55 wt%, based on a total weight of the complex absent any weight contributed by the carrier.

Complexed Active Ingredient (i.e., Active Ingredient in the Complex)

The active ingredient in the complex can include a therapeutic agent, a prophylactic agent, a diagnostic agent, or a nutritional agent. The active ingredient in the complex can be a water-soluble agent or a water-insoluble agent. In a particular embodiment, the active ingredient in the complex is a water-insoluble agent.

In an embodiment, the active ingredient in the complex can include a nutritional agent. The nutritional agent can include a carbohydrate, a fat, a mineral, a protein, a vitamin, or any combination thereof. The nutritional agent can be a water-soluble agent or a water-insoluble agent. In a particular embodiment, the nutritional agent is a water-insoluble agent.

In an embodiment, the nutritional agent includes a vitamin. The vitamin can include water-soluble vitamin or a fat-soluble vitamin. The water-soluble vitamin can include one or more forms of Vitamin B or one or more forms of vitamin C, or any combination of one or more forms of Vitamin B and one or more forms of Vitamin C. The fat-soluble vitamin can include one or more forms of Vitamin A, Vitamin D, Vitamin E, and Vitamin K, or any combination

In a particular embodiment, the primary active ingredient includes a fat-soluble vitamin. In a preferred embodiment, the fat-soluble vitamin includes a Vitamin D or a Vitamin K, Vitamin D being most preferred.

In an embodiment, the Vitamin D can include one or more of the several forms of Vitamin D that exist. The forms of Vitamin D can include Vitamin D1, Vitamin D2, Vitamin D3, Vitamin D4, and Vitamin D5. Vitamin D1 includes a mixture of ergocalciferol and lumisterol. Vitamin D2 includes ergocalciferol, made from ergosterol found in plants. Vitamin D3 includes cholecalciferol, made from the ultraviolet irradiation of 7-dehydrocholesterol in the skin. Vitamin D4 includes 22-dihydroergocalciferol. Vitamin D5 includes sitocalciferol, made from 7-dehydrocholesterol.

The two major forms of Vitamin D are Vitamin D2 and Vitamin D3. In an embodiment, the Vitamin D includes either Vitamin D2, Vitamin D3, or a combination thereof. In a preferred embodiment, the Vitamin D includes Vitamin D3.

Vitamin K refers to a family of compounds with a common chemical structure of 2-methyl-1,4-naphthoquinone. In an embodiment, the active ingredient in the complex can include at least one form of Vitamin K, such as a phylloquinone (referred to as vitamin K1) and a menaquinone (referred to as Vitamin K2).

In an embodiment, the active ingredient can be present in the complex in an amount of at least 0.05 wt%, or at least 0.1 wt%, or at least 0.2 wt%, based on a total weight of the complex. The active ingredient can be present in the complex in an amount of no greater than 10 wt%, or no greater than 8 wt%, or no greater than 6 wt%, based on a total weight of the complex.

### Forming the Complex

Forming the particulate material includes first forming the complex between the complexing agent and the active ingredient. This can be accomplished by combining the complexing agent and the active ingredient in a solution. In a particular embodiment, it can include forming a first solution and a second solution.

The first solution can include a major portion of the active ingredient. For example, the first solution can include the active ingredient in an amount of at least 32 wt%, or at least 34 wt%, or at least 36 wt%, based on a total weight of the first solution. As another example, the first solution can include the active ingredient in an amount of no greater than 65 wt%, or no greater than 60 wt%, or no greater than 55 wt%, based on a total weight of the second solution.

The second solution can include a major portion of the complexing agent. For example, the second solution can include the complexing agent in an amount of at least 35 wt%, or at least 40 wt%, or at least 45 wt%, based on a total weight of the second solution. As another example, the second solution can include the complexing agent in an amount of no greater than 80 wt%, or no greater than 70 wt%, or no greater than 60 wt%, based on a total weight of the second solution.

In a particular embodiment, the first solution can also include a minor portion of the complexing agent. For example, the first solution can include the complexing agent in an amount of at least 0.1 wt%, or at least 0.5 wt%, or at least 1 wt%. As another example, the first solution can include the complexing agent in an amount of no greater than 10 wt%, or no greater than 8 wt%, or no greater than 6 wt%.

In an embodiment, the first solution can also include a first solvent and the second solution can also include a second solvent. While the solvents can be helpful in forming the solutions to prepare the complex, all the solvents may be evaporated during a drying step later in the method.

The first solvent and the second solvent can be a protic solvent. The protic solvent of the first solvent can have a same formula or a different formula as compared to the protic solvent of the second solvent. In a particular embodiment, the first solvent has different formula as compared to the second solvent.

In an embodiment, the first solvent includes an organic solvent, water, or a combination thereof. For example, the first solvent can include a major portion of an organic solvent. The first solvent can include the organic solvent in an amount of at least 40 wt%, or at least 45 wt%, or at least 50 wt%. The first solvent can include the organic solvent in an amount of no greater than 80 wt%, or no greater than 70 wt%, or no greater than 60 wt%, based on a total weight of the first solution.

In an embodiment, the second solvent includes an organic solvent, water, or a combination thereof. For example, the second solvent can include a major portion of water. In a particular embodiment, the second solvent consists essentially of water. The second solution can include water in an amount of at least 35 wt%, or at least 40 wt%, or at least 45 wt%, based on a total weight of the second solution. As another example, the second solution can include water in an amount of no greater than 80 wt%, or no greater than 70 wt%, or no greater than 60 wt%, based on a total weight of the second solution.

The organic solvent can include an alcohol. The alcohol can include an ethanol, a methanol, a glycerol, or any combination thereof. In an embodiment, the organic solvent includes an ethanol. In a particular embodiment, the organic solvent includes an absolute ethanol.

The first solution can be added to the second solution. The second solution can be added to the first solution. The first solution and the second solution can be added to a same container simultaneously or contemporaneously. The first solution and the second solution combined form a complex solution that includes the complex.

### Carrier

Once the complex is formed in solution, a carrier can then be added to form the particulate material. In a particular embodiment, the carrier can be added to the complex by a mixing step.

In an embodiment, the carrier can include a calcium carbonate, a microcrystalline cellulose, a calcium phosphate, a disaccharide, a sugar alcohol, a polysaccharide, or any combination thereof. In an embodiment, the calcium carbonate can include a light type, a heavy type, or a spray-dry type calcium carbonate carrier particulate. In an embodiment, the calcium phosphate can include a calcium hydrogen phosphate or a calcium hydrogen phosphate particulate. In an embodiment, the disaccharide can include a lactose or a sucrose particulate. In an embodiment, the polysaccharide can include a starch particulate.

The carrier can be present in the particulate material in an amount of at least 55 wt%, or at least 60 wt%, or at least 65 wt%, based on a total weight of the particulate material, particularly when the carrier is a less dense material such as microcrystalline cellulose. Where the carrier is a denser material such as calcium carbonate, the carrier can be present in the complex particle in an amount of at least 70 wt%, or at least 75 wt%, or at least 80 wt%, based on a total weight of the particulate material.

In a particular embodiment, the carrier can be comprised of the same material as an active ingredient. For example, the carrier can comprise a calcium carbonate where an active ingredient in the formulation is also calcium carbonate. This is preferred, because it avoids the addition of excipients that have no immediate benefit for consumer. In another embodiment, the carrier can be comprised of a material that is different than an active ingredient. For example, the carrier can comprise a microcrystalline cellulose where microcrystalline cellulose is not an active ingredient in the formulation. This may be preferred namely when the particulate material is used to make tablets at a later stage.

The mixing step combining the complex and the carrier may include granulation. The granulation can include wet granulation or fluidized bed granulation. The wet granulation can include high shear wet granulation.

In the fluidized bed granulation, the carrier is in the form of particulates that are suspended in an air stream while a liquid containing the complex solution is sprayed. The suspended carrier particulates form complex granules by interfacing with the complex solution in the liquid spray and aggregating with other carrier particulates.

As discussed previously, the method can include a drying step. When wet granulation is used, a separate fluidized bed drying step can follow the wet granulation step. When a fluidized bed is used for granulation, the drying step may take place in the same fluidized bed. For example, granulation process can be a one-step process including fluidized bed granulation and drying.

The granulation and drying process described herein can provide a yield of at least 85%, or at least 90%, or at least 95%. A higher yield may occur when the fluidized bed is used for granulation and drying as it is a one-step process rather than transferring from one format to another.

The particulate material can include a plurality of particulates, such as a plurality of granules, where the particulates contain the complex. In an embodiment, the particulates (e.g., granules) of the particulate material (e.g., granulated complex) contain the complex and the carrier.

Applicant has discovered that the use of a carrier in this method provides a surprisingly significant improvement in active ingredient content uniformity, particularly when the active ingredient is not readily soluble on its own (i.e., when not in a complex). Content uniformity is an indicator for content distribution. A smaller value would indicate a better mixing. In an embodiment, the content uniformity between particulates of the particulate material is no greater than 2%, or no greater than 1.7%, or no greater than 1.4%. In a particular embodiment, the content uniformity between the particulates of the particulate material is no greater than 1.2%, or no greater than 1%, or no greater than 0.8%.

Applicant has discovered that the particulate material formed according to an embodiment of the method disclosed herein contains fewer impurities than a commercially available form of the active ingredient that is not in a complex or has not been formed using a carrier.

Applicant has discovered that the particulate material formed according to an embodiment of the method disclosed herein exhibits improved stability as compared to a commercially available form of the active ingredient that is not in a complex or has not been formed using a carrier. Surprisingly, the improved stability is exhibited in a variety of environments, such as at 40 deg C at 75% humidity over 6 months and at 25 deg C at 60% humidity over 24 months.

As discussed previously, the complex includes an active ingredient and a complexing agent. In an embodiment, the active ingredient is better suited to provide the beneficial effect as an ingredient complex as compared to the active ingredient that is not in a complex.

### Formulation

In certain embodiments, the complex can be added to a formulation. The formulation can be manufactured in a dosage form. The dosage form including the complex can be delivered to the body.

The formulation can include a complementary active in addition to the active ingredient in the complex. The complementary active can include any of the ingredients listed above with respect to the active ingredient in the complex. The complementary active is different than the active ingredient in the complex.

In a particular embodiment, the complementary active can be the same component as the carrier. For example, the carrier can be a calcium carbonate, as discussed above, and the active can also be a calcium carbonate. Calcium carbonate can function as a carrier as well as provide relief with respect to heartburn, acid indigestion, sour stomach, and upset stomach associated with these symptoms.

In a more particular embodiment, the formulation includes a calcium carbonate carrier in the complex and the complementary active also includes calcium carbonate, the same component as the carrier. For example, the complex can include a Vitamin D as the active ingredient, a cyclodextrin as the complexing agent, and a calcium carbonate as both the carrier and the complementary active. Vitamin D, administered in combination with calcium, can make it possible to increase the absorption of the calcium. Having the carrier be the same component as the complementary active can provide some benefits, such as ease in processing.

On the other hand, in another embodiment, the complementary active is not the same component as the carrier. For example, the carrier can be microcrystalline cellulose and the complementary active can include a calcium carbonate.

The formulation can further include one or more excipients. Non-limiting examples of an excipient include any and all solvents, dispersion media, diluents, or other liquid vehicles, dispersion or suspension aids, surface active agents, isotonic agents, thickening or emulsifying agents, and preservatives.

Excipients known by those skilled in the art may be useful in the practice of the present invention. Such excipients may include, but are not limited to, humectants such as propylene glycol, defoaming agents, buffers, electrolytes, preservatives such as sodium benzoate and disodium edetate, antioxidants, taste masking agents and various flavoring and coloring agents, for example.

In an embodiment, the formulation can include the complex described herein, a complementary active, and an excipient, including a filler, a sweetener, a binder, and a flavoring agent. The filler can be present in an amount of at least 20 wt%, or at least 25 wt%, or at least 30 wt%, or no greater than 60 wt%, or no greater than 55 wt%, or no greater than 50 wt%, based on a total weight of the formulation. The sweetener can be present in an amount of at least 6 wt%, or at least 8 wt%, or at least 10 wt%, or no greater than 40 wt%, or no greater than 35 wt%, or no greater than 30 wt%. The binder can present in an amount of at least 0.1 wt%, or at least 0.3 wt%, or at least 0.5 wt%, or no greater than 11 wt%, or no greater than 9 wt%, or no greater than 7 wt%. The flavoring agent can be present in an amount of at least 0.1 wt%, or at least 0.3 wt%, or at least 0.5 wt%, or no greater than 10 wt%, or no greater than 8 wt%, or no greater than 6 wt%.

In a particular embodiment, the formulation can include the formulation described in Table 1 below.

**Table 1**

| **Component** | **% w/w** |
|---|---|
| Active Complex | 0.40 - 50 |
| Complementary active | 2 - 50 |
| Filler | 30 - 50 |
| Sweetener | 10 - 30 |
| Binder | 0.5 - 7 |
| Flavor and Coating | 1 - 6 |

In an embodiment, the formulation can be applied in various dosage forms. In an embodiment, the formulation can be applied in a tablet dosage form, a granule dosage form, a solution dosage form, a softgel dosage form, a gummy dosage form, a jelly dosage form, or any combination thereof.

In yet another aspect, a method of treating calcium deficiency in a mammalian subject comprises administering a therapeutically effective amount of any composition described herein to a mammalian subject in need thereof.

In yet another aspect, a method of treating Vitamin D deficiency in a mammalian subject comprises administering a therapeutically effective amount of any composition described herein to a mammalian subject in need thereof.

### LIST OF EMBODIMENTS

The skilled artisan would understand that modifications and variations of the compositions described herein can be made within the scope of the invention. The following exemplary embodiments illustrate some, but not all variations of the invention.
Embodiment 1. A method of making a particulate material, comprising forming a complex from a water-insoluble vitamin and a complexing agent comprising a cyclodextrin, and forming a particulate material from the complex, wherein the particulate material comprises a plurality of particulates and the content uniformity of the plurality of particulates is no greater than 2%.
Embodiment 2. The method of claim 1, further comprising the step of combining the complex with a carrier to form the particulate material.
Embodiment 3. The method of embodiment 2, wherein the carrier comprises a calcium carbonate, a microcrystalline cellulose, a calcium phosphate, a disaccharide, a sugar alcohol, a polysaccharide, or any combination thereof.
Embodiment 4. The method of embodiment 3, wherein the carrier comprises a calcium carbonate.
Embodiment 5. The method of embodiment 3, wherein the carrier comprises microcrystalline cellulose.
Embodiment 6. The method of any one of the preceding claims, wherein the cyclodextrin is a substituted cyclodextrin.
Embodiment 7. The method of embodiment 6, wherein the substituted cyclodextrin comprises a hydroxypropyl-substituted cyclodextrin.
Embodiment 8. The method of any one of the preceding claims, wherein the complexing agent comprises a hydroxypropyl-β-cyclodextrin.
Embodiment 9. The method of any one of the preceding claims, wherein the water-insoluble vitamin comprises a Vitamin A, a Vitamin D, a Vitamin E, a Vitamin K, or any combination thereof.
Embodiment 10. The method of embodiment 9, wherein the water-insoluble vitamin is a Vitamin D.
Embodiment 11. The method of embodiment 9, wherein the water-insoluble vitamin is a Vitamin D3.
Embodiment 12. The method of embodiment 9, wherein the water-insoluble vitamin is a Vitamin K.
Embodiment 13. The method of embodiment 9, wherein the water-insoluble vitamin is a Vitamin K2.
Embodiment 14. The method of any of the preceding claims, wherein forming the complex comprises providing a first solution comprising the water-insoluble vitamin and a second solution comprising the cyclodextrin, and combining the first and second solution to form the complex.
Embodiment 15. The method of embodiment 14, wherein the first solution further comprises a minor portion of the complexing agent.
Embodiment 16. The method of embodiment 2, wherein combining the complex and the carrier is performed using wet granulation or fluidized bed granulation.
Embodiment 17. The method of embodiment 2, wherein fluidized bed granulation is used to combine the complex and the carrier.
Embodiment 18. The method of any one of embodiments 2 to 24, wherein the content uniformity of the plurality of particulates is no greater than 2%.
Embodiment 19. The method of any one of the preceding claims, wherein the particulate material comprises a plurality of particulates and the particulates are granules.
Embodiment 20. A particulate material formed according to the method of any one of the preceding claims.
Embodiment 21. A particulate material comprising a complex and a carrier, the complex comprising a cyclodextrin and a fat soluble vitamin.
Embodiment 22. The particulate material of embodiment 27, wherein the fat soluble vitamin comprises Vitamin D.
Embodiment 23. The particulate material of embodiment 28, wherein the fat soluble vitamin comprises Vitamin D3.
Embodiment 24. The particulate material of embodiment 27, wherein the fat soluble vitamin comprises Vitamin K.
Embodiment 25. The particulate material of embodiment 30, wherein the fat soluble vitamin comprises Vitamin K2.
Embodiment 26. The particulate material of embodiment 21, wherein the carrier comprises calcium carbonate or microcrystalline cellulose.
Embodiment 27. A dosage form comprising the particulate material of any one of embodiments 20-26.
Embodiment 28. The dosage form of embodiment 27, further comprising a complementary active ingredient.
Embodiment 29. The dosage form of any one of embodiments 27 to 28, wherein the complementary active ingredient comprises calcium carbonate.
Embodiment 30. The dosage form of any one of embodiments 27 to 29, further comprising an excipient.
Embodiment 31. The dosage form of any one of embodiments 27 to 30, wherein the dosage form comprises a tablet dosage form, a granule dosage form, a solution dosage form, a softgel dosage form, a gummy dosage form, a jelly dosage form, or any combination thereof.
Embodiment 32. The dosage form of any one of embodiments 27 to 31, wherein the dosage form is a tablet.
Embodiment 33. The dosage form of any one of embodiments 27 to 32, wherein the dosage form has a %LC drop of less than 5% when subjected to an accelerated stability test.
Embodiment 34. The dosage form of any one of embodiments 27 to 33, wherein the dosage form has a %LC drop of less than 5% when subjected to a long term stability test.

### EXAMPLES

Some embodiments described herein are further illustrated in the following non-limiting examples.

### EXAMPLE 1

### Content Uniformity

Two sample granules (Samples 1 and 2) contained Vitamin D in Granulated Complex 1, which was a HPβCD/VD3 complex as described herein where the carrier used was CaCO₃. Granulated Complex 1 was made according to the formulation set forth in Table 2. Three control granules (Controls 1, 2, and 3) also contained Vitamin D but from a commercially available source (VD3 CWS 100) that does not contain the complexing agent.

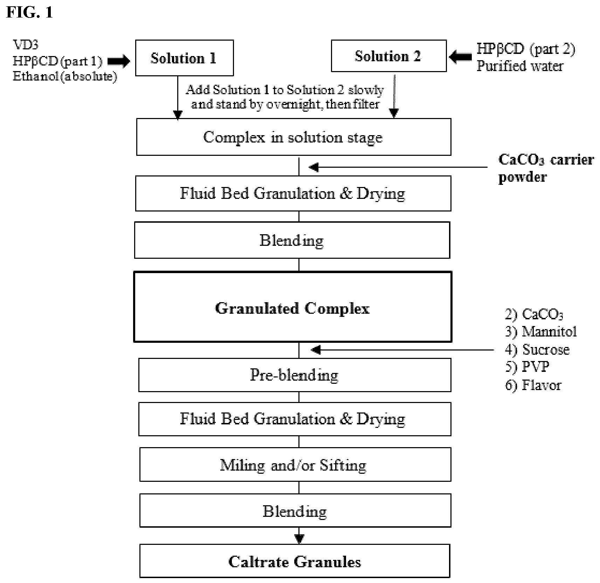

Samples 1 and 2, and Controls 1, 2, and 3, were made according to the formulas set forth in Table 3, and Samples 1 and 2 were made according to the method set forth in the flow chart of FIG. 1. Sample 1 and Control 1 were made as a small batch (2kg), while Sample 2 and Control 2 and 3 were made as large batch (250kg). While Control 2 and 3 were made according to the same formula, Control 2 had granules with a mesh size of 60, and Control 3 had granules with a mesh size of 80.

**Table 2 - Granulated Complex 1**

| **S.No** | **Name** | **Function** | **% w/w** |
|---|---|---|---|
| **4%D₃/39%HPβCD ethanol solution (Solution 1)** | | | |
| **1** | Vitamin D3 | Active | 0.03 |
| **2** | HPβCD (Part 1) | Complexing agent | 0.32 |
| **3** | Ethanol (absolute) | Solvent * | 0.47 |

| **50% HPβCD water solution (Solution 2)** | | | |
|---|---|---|---|
| **4** | HPβCD (Part 2) | Complexing agent | 7.54 |
| **5** | Purified water | Solvent* | 7.54 |

| **Carrier for complex-1 granulation** | | | |
|---|---|---|---|
| **6** | CaCO₃ powder | Carrier | 84.09 |

| | | | |
|---|---|---|---|
| **All the solvents will be evaporated during the drying process* | | | |

**Table 3 - Samples and Controls**

| | **2 kg** | | **250 kg** | | |
|---|---|---|---|---|---|
| **Component** | **Sample 1 (kg)** | **Control 1 (kg)** | **Sample 2 (kg)** | **Control 2 (kg)** | **Control 3 (kg)** |
| CaCO3 | 0.8333 | 0.8333 | 104.167 | 104.167 | 104.167 |
| Granulated Complex 1 | 0.01047 | -- | 1.311 | -- | -- |
| Commercially Available Vitamin D Source | -- | 0.00147 | -- | 0.197 | 0.197 |
| Mannitol | 0.6551 | 0.6551 | 80.772 | 81.887 | 81.887 |
| Sucrose | 0.3500 | 0.3500 | 43.750 | 43.750 | 43.750 |
| Polyvinylpyrrolidone (PVP) | 0.1000 | 0.1000 | 12.500 | 12.500 | 12.5 |
| Flavoring | 0.0600 | 0.0600 | 7.500 | 7.500 | 7.500 |

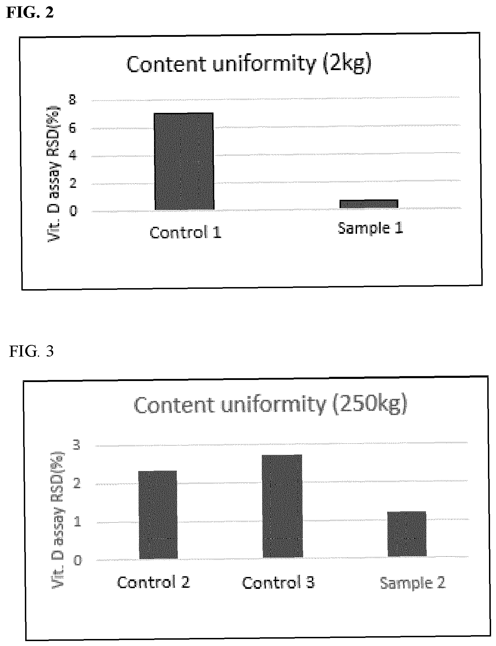

The Samples and Controls were subjected to the Content Uniformity test. The Content Uniformity Test is a standard Vitamin D assay known to one of ordinary skill in the art. The results of the Content Uniformity Test are provided in FIGs. 2 and 3, wherein RSD is the standard deviation. Content uniformity is an indicator for content distribution, and a smaller value would indicate better content distribution. Content uniformity was measured according to the standards of Chinese Pharmacopeia. As seen in FIGs. 2 and 3, Sample 1 (small batch, 2 kg) and Sample 2 (large batch, 250 kg) both had an improved Content Uniformity as compared to Controls 1, 2, and 3.

### EXAMPLE 2

### Stability

Two sample tablets (Samples 3 and 4) contained Vitamin D in Granulated Complex 2, which was a HPβCD/VD3 complex as described herein where the carrier used was microcrystalline cellulose (MCC). Granulated Complex 2 was made according to the formulation set forth in Table 4. Two control tablets (Controls 4 and 5) also contained Vitamin D but from a commercially available source that does not contain the complexing agent.

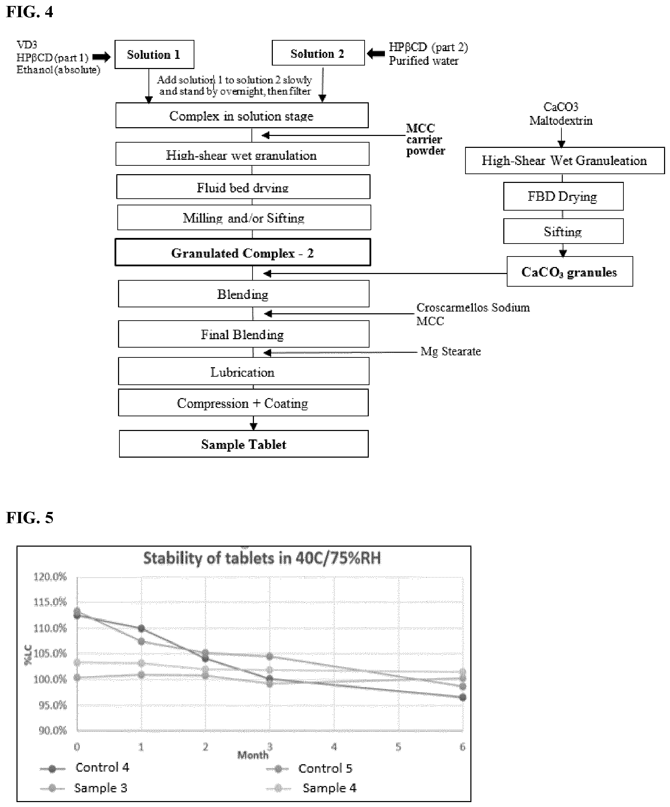

Samples 3 and 4, and Controls 4 and 5, were made according to the formulas set forth in Table 5 and the method set forth in the flowchart of FIG. 4. Samples 3 and 4 were prepared with a Vitamin D overage of 5%, whereas Controls 4 and 5 were prepared with Vitamin D overage of 15%.

**Table 4 - Granulated Complex 2**

| **S.No** | **Name** | **Function** | **% w/w** |
|---|---|---|---|
| **4%D₃/39%HPβCD ethanol solution (Solution 1)** | | | |
| **1** | Vitamin D3 | Active | 0.07 |
| **2** | HPβCD (Part 1) | Complexing agent | 0.63 |
| **3** | Ethanol (absolute) | Solvent* | 0.92 |

| **50% HPβCD water solution (Solution 2)** | | | |
|---|---|---|---|
| **4** | HPβCD (Part 2) | Complexing agent | 14.70 |
| **5** | Purified water | Solvent* | 14.70 |

| **Carrier for complex-2 granulation** | | | |
|---|---|---|---|
| **6** | Microcrystalline cellulose powder | Carrier | 68.97 |

| | | | |
|---|---|---|---|
| **All the solvents will be evaporated during the drying process* | | | |

**Table 5 - Samples and Controls**

| **Component** | **Sample 3 (mg/tablet)** | **Sample 4 (mg/tablet)** | **Control 4 (mg/tablet)** | **Control 5 (mg/tablet)** |
|---|---|---|---|---|
| CaCO3 | 1579 | 1579 | 1579 | 1579 |
| Granulated Complex 2 | 4.3 | 1.9 | - | - |
| Commercially Available Vitamin D Source | - | - | 2.0 | 2.0 |
| Microcrystalline Cellulose | 33 | 35 | 80 | 80 |
| Lactose monohydrate | | | 30 | 30 |
| Hydroxy propyl methyl cellulose | | | 66 | 66 |
| Croscarmellose sodium | 20 | 20 | | |
| Sodium methyl starch | | | 8.8 | 8.8 |
| Soldium lauryl sulphate | - | - | | |
| Magnesium stearate | 4 | 4 | 8.8 | 8.8 |
| Tablet coating | 30 | 30 | 30 | 30 |

The Samples and Controls were subjected to an Accelerated Stability Test and a Long Term Stability Test. The Accelerated Stability test subjected each of the tablets to an environment having a temperature of 40°C and a relative humidity of 75% for a period of six months, where a standard analytical procedure was used to measure the stability of the Vitamin D in each tablet. The Long Term Stability Test subjected each of the tablets to an environment having a temperature of 25°C and a relative humidity of 60% for a period of 25 months, where a standard analytical procedure was used to measure the stability of the Vitamin D in each tablet.

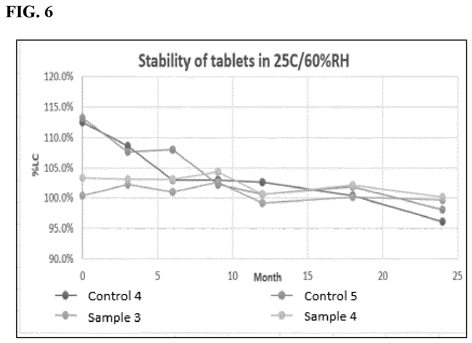

The results of the Accelerated Stability Test are provided in FIG. 5, and the results of the Long Term Stability Test are provided in FIG. 6.

As seen from FIGs. 5 and 6, the Samples and Controls at T0 all exhibited an acceptable drop of less than 5% of the label claim (%LC) after processing steps. However, Samples 2 and 3 outperformed Controls 4 and 5 in both Accelerated and Long Term stability. Samples 2 and 3 exhibited a drop in (%LC) of less than 5%, whereas Controls 4 and 5 exhibited a drop of greater than 10%. Despite both control tablets starting with a significantly greater %LC at T0 as compared to the Sample tablets, the %LC for the control tablets dropped below %LC for the sample tablets by the end of each test. Meanwhile, Samples 2 and 3 were able to maintain their %LC within 5% and with minimal change throughout both tests. This result indicated an unexpected increase in stability in tablets prepared using the Vitamin D complex described herein, as compared to tablets prepared using a commercially available Vitamin D source.

## Claims

1. A method of making a particulate material, comprising forming a complex from a water-insoluble vitamin and a complexing agent comprising a cyclodextrin, and forming a particulate material from the complex, wherein the particulate material comprises a plurality of particulates and the content uniformity of the plurality of particulates is no greater than 2%.

2. The method of claim 1, further comprising the step of combining the complex with a carrier to form the particulate material.

3. The method of claim 2, wherein the carrier comprises a calcium carbonate, a microcrystalline cellulose, a calcium phosphate, a disaccharide, a sugar alcohol, a polysaccharide, or any combination thereof, preferably a calcium carbonate.

4. The method of any one of the preceding claims, wherein the cyclodextrin is a substituted cyclodextrin, preferably comprising a hydroxypropyl-β-cyclodextrin.

5. The method of any one of the preceding claims, wherein the water-insoluble vitamin comprises a Vitamin A, a Vitamin D, a Vitamin E, a Vitamin K, or any combination thereof.

6. The method of claim 5, wherein the water-insoluble vitamin is a Vitamin D, preferably Vitamin D3.

7. The method of any of the preceding claims, wherein forming the complex comprises providing a first solution comprising the water-insoluble vitamin and a second solution comprising the cyclodextrin, and combining the first and second solution to form the complex, wherein the first solution further comprises less than 30%, or less than 25%, or less than 20% of the complexing agent.

8. The method of claim 2, wherein combining the complex and the carrier is performed using wet granulation or fluidized bed granulation.

9. A particulate material formed according to the method of any one of the preceding claims.

10. A particulate material comprising a complex and a carrier, the complex comprising a cyclodextrin, preferably hydroxypropyl-β-cyclodextrin, and a fat soluble vitamin, preferably Vitamin D3.

11. The particulate material of claim 10, wherein the carrier comprises calcium carbonate or microcrystalline cellulose.

12. A dosage form comprising the particulate material of any one of claims 9 to 11, further comprising a complementary active ingredient, wherein the complementary active ingredient comprises calcium carbonate.
